# EUROPEAN PATENT SPECIFICATION

(11) **EP 3 421 057 B1**
(45) Date of publication and mention of the grant of the patent: **17.02.2021**
(21) Application number: 18180713.2
(22) Date of filing: 29.06.2018
(51) Int. Cl.: A61L 27/36, A61L 27/38, A61L 27/60, A61F 2/10, C12N 5/071, A61L 27/24

(54) **PRODUCING METHOD OF ARTIFICIAL SKIN AND ARTIFICIAL SKIN**
VERFAHREN ZUR HERSTELLUNG VON KÜNSTLICHER HAUT UND KÜNSTLICHE HAUT
PROCÉDÉ DE PRODUCTION DE PEAU ARTIFICIELLE ET PEAU ARTIFICIELLE

(30) Priority: 30.06.2017 KR 20170083516
(43) Date of publication of application: 02.01.2019
(73) Proprietor: Amorepacific Corporation, Seoul 04386 (KR)
(72) Inventor: LEE, Sung Hoon, Gyeonggi-do (KR); BAE, Il-Hong, Gyeonggi-do (KR); MIN, Daejin, Gyeonggi-do (KR); OH, Soojung, Gyeonggi-do (KR); LEE, Tae Ryong, Gyeonggi-do (KR); CHO, Eun gyung, Gyeonggi-do (KR)
(74) Representative: Brevalex

(56) References cited:
- EP-A1- 3 072 535
- WO-A1-2005/007835
- WO-A1-2015/166455
- WO-A1-2016/151134

## Description

### CROSS-REFERENCE TO RELATED APPLICATION

This application claims priority to and the benefit of Korean Patent Application No. 10-2017-0083516 filed in the Korean Intellectual Property Office on June 30, 2017.

### BACKGROUND OF THE INVENTION

### (a) Field of the Invention

This disclosure relates to a method of producing artificial skin. This disclosure specifically relates to a method of producing artificial skin having skin pigmentation. In addition, this disclosure relates to artificial skin including keratinocytes and a melanocytes.

### (b) Description of the Related Art

Skin is an organ covering the outside of a body, and consists of three layers of an epidermis, a dermis, and a hypodermis from the outmost thereof. The epidermis is mostly composed of keratinocytes of a stratified squamous epithelium. The dermis formed of a matrix protein such as a collagen fiber and an elastic fiber is present beneath the epidermis, and includes blood vessels, nerves, sweat glands, and the like. The hypodermis is formed of adipocytes. The skin maintains its shape through an interaction of the aforementioned various cells and their substances, and performs various functions of adjusting a body temperature, working as a barrier against an external environment, and the like.

The skin may be three-dimensionally restructured as artificial skin by using skin cells and a skin substance such as collagen, elastin, and the like, and the artificial skin is formed of living fibroblasts and keratinocytes and thus shows similar structural and functional characteristics to real skin and accordingly is called reconstructed skin (a skin equivalent). The artificial skin is mainly a polymer composite showing equivalent properties to skin in terms of elasticity, strength, material permeability, and the like, but is different therefrom in terms of not showing life like real skin. The artificial skin may not only be used for replacement (a permanent engrafting type) or reproduction (a temporary covering type), but may also be used in various fields such as skin physiology research, skin stimulus evaluation, skin effect evaluation, and the like.

EP 3 072 535 A1 discloses a method of producing a skin substitute comprising mixing fibroblasts and collagen to form a dermis simulating layer; applying keratinocytes and melanocytes on the dermis simulating layer and culturing the same; culturing the dermis simulating layer submersed for 8-24h in a first medium that comprises DMEM:F12 3:1, 10%FCS (i.e. a keratinocyte medium); and interface culturing for 7 days the dermis simulating layer that is cultured in the first medium in a second medium that is DMEM/F12/10%FCS, comprising HA; interface culturing for 7 days the dermis simulating layer that is cultured in the first medium in a second medium that is DMEM/F12/10%FCS, comprising HA, ascorbic acid and penicillin-streptomycin.

WO 2016/151134 A1 discloses a method of producing a skin substitute comprising mixing fibroblasts and collagen to form a dermis simulating layer; applying keratinocytes and melanocytes on the dermis simulating layer and culturing the same; culturing the dermis simulating layer submersed for 8-24h in a first medium that comprises DMEM:F12 3:1, 10%FCS (i.e. a keratinocyte medium); and interface culturing for 7 days the dermis simulating layer that is cultured in the first medium in a second medium that is DMEM/ F12/10%FCS, comprising HA; interface culturing for 7 days the dermis simulating layer that is cultured in the first medium in a second medium that is DMEM/ F12/10%FCS, comprising HA, ascorbic acid and penicillin-streptomycin.

A pigmentation-induced artificial skin model may be, for example, used for a whitening effect evaluation of cosmetics. Pigmentation of skin is mainly caused by presence of a melanic pigment in the epidermis, and melanin is synthesized by melanocytes, which are dendritic cells in a basal layer of the epidermis. A conventional pigmented artificial skin is formed by using a method of separately culturing keratinocytes and melanocytes, and then co-seeding them to induce development of an epidermis layer.

### SUMMARY OF THE INVENTION

The method of separately culturing keratinocytes and melanocytes and co-seeding them to form an epidermis layer may complicate a process of forming artificial skin. In addition, since conditions for substantially culturing keratinocytes and melanocytes together are difficult to set, and thus the melanocytes are not wholly present in a final artificial skin model, the artificial skin model may not function as a normal pigmented artificial skin model.

Accordingly, a production method of artificial skin according to claim 1 provides a more similar pigmented artificial skin to real pigmented skin by using keratinocytes.

The second medium may contain a component inducing differentiation of the melanocyte stem cells.

The first medium may be disposed to contact a bottom surface of the dermis simulating layer, and an opposite surface to the bottom surface where the first medium is disposed may be exposed to the air.

The culturing in the second medium may proceed directly after the culturing in the first medium.

The culturing in the first and second media may be performed within 20 days in total.

The culturing period in the first medium may be equal to or longer than the culturing period in the second medium.

The second medium may be prepared by mixing the DMEM medium and the F12 medium in a ratio of about 1:1 to about 4:1.

The second medium may further include at least one selected from hydrocortisone, triiodothyronine, cholera toxin, ascorbic acid, and calcium chloride (CaCl₂).

The culturing in the second medium may proceed in an exposure state to the air.

Another embodiment provides artificial skin including a dermis simulating layer and an epidermis simulating layer on the dermis simulating layer, wherein the epidermis simulating layer includes an epidermis basal layer part being adjacent to the dermis simulating layer and a horny layer part exposed to the air, and keratinocytes present in the horny layer and melanocytes present in the epidermis basal layer originate from the same person.

Still another embodiment provides a method of evaluating an effect of a pigmentation controlling agent, which includes applying the pigmentation controlling agent to artificial skin formed according to the aforementioned production method or artificial skin having the aforementioned structure and comparing a pigmentation degree of the artificial skin to which the pigmentation controlling agent is applied with a pigmentation degree of artificial skin to which the pigmentation controlling agent is not applied.

The pigmentation controlling agent may be applied through direct contact with the artificial skin.

The production method according to an embodiment may induce differentiation of a melanocyte stem cell present among keratinocytes and thus provide pigmented artificial skin without using the melanocyte. Artificial skin according to the production method is similar to real skin and thus may provide improved reliability of evaluation data when used as a skin whitening evaluation model.

### BRIEF DESCRIPTION OF THE DRAWINGS

FIG. 1 is a flowchart illustrating a production method of artificial skin according to an embodiment,
FIG. 2 is a microscope photograph showing a cross-section of artificial skin according to Example 1 after dying H&E tissues, and
FIG. 3 is a microscope photograph showing a cross-section of artificial skin according to Comparative Example 1 after dying H&E tissues.

### DETAILED DESCRIPTION OF THE EMBODIMENTS

Hereinafter, exemplary embodiments of the present disclosure will be described in detail, and may be easily performed by a person having ordinary skill in the related art. However, this disclosure may be embodied in many different forms, and is not to be construed as limited to the exemplary embodiments set forth herein.

In the present specification, "artificial skin" is skin that is three-dimensionally reconstructed by using skin cells and a skin substance such as collagen and the like, and includes any polymer composite showing similar structural and functional characteristics to those of real skin.

In the present specification It will be understood that when an element such as a layer, film, region, or substrate is referred to as being "on" another element, it may be directly on the other element or intervening elements may also be present. In contrast, when an element is referred to as being "directly on" another element, there are no intervening elements present.

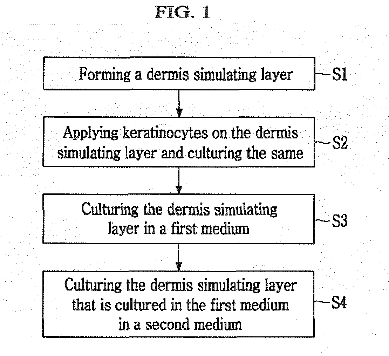

Hereinafter, the production method of artificial skin according to an embodiment is illustrated with reference to FIG. 1.

FIG. 1 is a flowchart illustrating the production method of artificial skin according to an embodiment.

Referring to FIG. 1, a method of producing artificial skin according to an embodiment includes forming a dermis simulating layer (S1), applying keratinocytes on the dermis simulating layer and culturing the same (S2), culturing the dermis simulating layer in the first medium (S3), and culturing the dermis simulating layer that is cultured in the first medium in a second medium (S4).

First, the formation of a dermis simulating layer (S1) is illustrated.

A dermis is a skin layer beneath an epidermis formed of connective tissues, and thus works as a buffer and protects a body from pressure as well as stresses and strains. The dermis is firmly connected with the epidermis through a basement membrane. The dermis provides a substrate for supporting various components such as blood vessels, nerves, and the like that are inherent in the structure.

The dermis simulating layer is formed by using a mixed material of fibroblasts and collagen in with a ratio that is correlated with that of real skin. The dermis simulating layer may consist of at least two layers, for example, a first dermis layer containing the collagen and a second dermis layer containing the fibroblasts. In this case, a dermis contraction phenomenon of the artificial skin may be further alleviated.

The first dermis layer may contain collagen as well as an extracellular matrix such as elastin, chitosan, glycosaminoglycans (GAGs), and hyaluronic acid (HA).

The second dermis layer may be present on the first dermis layer, and contains fibroblasts as well as an extracellular matrix such as elastin, chitosan, glycosaminoglycans (GAGs), and hyaluronic acid (HA).

The collagen may originate from natural collagen such as that from a cow, a rat tail, or a fish, or may be produced through genetic engineering, and may contract under presence of fibroblasts.

The dermis simulating layer may generally have a thickness of greater than or equal to about 0.05 cm, and particularly, in a range from about 0.05 to about 2 cm, but the thickness may be increased or decreased, as long as it produces no damage to advantageous characteristics of the artificial skin according to the present invention.

As aforementioned, the dermis simulating layer may be, for example, cultured for about 5 days to about 9 days, about 6 days to about 8 days, or about 7 days, after being formed by the mixed material of fibroblasts and collagen.

When the dermis simulating layer is formed, keratinocytes are applied thereon, and the dermis simulating layer is cultured (S2).

The keratinocytes constitute about 80 % to about 90 % of cells of the epidermis layer, are formed through mitosis in the basal layer, which is the deepest layer of the epidermis layer, and then rise toward a skin surface.

The culturing after applying the keratinocytes (S2) is a first step for forming an epidermis simulating layer of the artificial skin, wherein the keratinocytes may, for example, originate from a human. The keratinocytes may be any commercially available keratinocytes, which may not only be directly separated from a human or cultured after separation, but may also be induced from a different cell from a human cell. The commercially available human keratinocytes may include NHEK-Neo, Pooled (Neonatal Normal Human Epidermal Keratinocytes, Pooled: Product No. 00192906, Tissue registration No. P867, white people), NHEK-Neo (Product No. 00192907, Tissue registration No. 20647, white people), NHEK-Adult (Product No. 00192627, Tissue registration No. 21155, white people), NHEK-Neo (Product No. 00192907, Tissue registration No. 18080, black people), which are provided by Lonza. For example, the commercially available human keratinocytes may include HEKn (Human Epidermal Keratinocytes, neonatal: Product No. C0015C, Tissue registration No. 1781129, white people), and HEKn (Product No. C0015C, Tissue registration No. 1803827, black people), which are provided by Thermo Fisher Scientific Company. In order to maintain a keratinocyte's nature of growing while adhered to a basement membrane, a dish may be coated with about 0.1 to 0.2 % gelatin or about 0.1 to 0.2 mg/ml collagen type IV. The cells may be cultured in an incubator at about 35 to 37 °C under about 5 to10 % CO₂ and sub-cultured when density thereof reaches about 70 to 80 %.

The keratinocytes may be, for example, applied on the dermis simulating layer through seeding, and the culture may, for example, proceed for about 1 day to about 4 days, about 1 day to about 3 days, or about 1 day to about 2 days, but is not limited thereto.

Among the keratinocytes, melanocyte stem cells may be included in a small amount. The melanocyte stem cells are differentiated into melanocytes through culturing in first and second media, which will be described later.

After the application and culturing of keratinocytes (S2), the cells are cultured in the first medium, which is a keratinocyte medium (S3).

Herein, the "keratinocyte medium" denotes a medium for culturing human keratinocytes, and can be any medium known in the related art. For example, the keratinocyte medium may include a bovine pituitary extract (BPE), a human epidermal growth factor (hEGF), bovine insulin, hydrocortisone, gentamicin, and amphotericin-B (GA-1000). In addition, epinephrine and transferrin may be further added to the components. The medium may be a commercially available one, for example, CnT-3D-PR (CellnTEC Advanced Cell Systems AG).

The first medium may be disposed to contact a bottom surface of the dermis simulating layer, and an opposite surface to the bottom surface of the first medium is disposed may be exposed to the air. The culture in the first medium may, for example, proceed for about 3 days to about 10 days, about 4 days to about 10 days, about 5 days to about 9 days, or about 4 days to about 8 days, but is not limited thereto.

Subsequently, the dermis simulating layer cultured in the first medium is cultured in a second medium including a DMEM medium and an F12 medium (S4).

This mixed medium may be prepared by mixing commercially available DMEM medium and F12 medium, for example, in a ratio of about 1:1 to about 4:1, and specifically about 2:1 to about 3:1, but is not limited thereto. The DMEM (Dulbecco's modified Eagle medium) is a medium for culturing a generally-used animal cell, and may include amino acids, vitamins, inorganic salts, glucoes, lipids, indicators, serums, and the like.

The second medium may include 100 % of the DMEM medium and the F12 medium based on a total amount of a culture medium, but additionally, it may include at least one selected from hydrocortisone, triiodothyronine, cholera toxin, ascorbic acid, and calcium chloride (CaCl₂).

The culturing in the second medium may proceed in an exposure state to the air like the culturing in the first medium.

The culturing in the second medium may subsequently proceed, for example, for about 4 days to about 15 days, about 5 days to about 13 days, about 6 days to about 10 days, or about 7 days to about 9 days, directly after the culturing in the first medium, but is not limited thereto. However, the culturing periods in the first and second media may be adjusted, for example, within about 20 days. For example, the culturing period in the first medium may be equal to or longer than the culturing period in the second medium.

After S2 to S4, an artificial skin structure having the epidermis simulating layer on the dermis simulating layer is obtained.

The production method of artificial skin according to an embodiment may differentiate melanocyte stem cells present among keratinocytes seeded on the dermis simulating layer before differentiating melanocytes through the sequential culturing in the first medium (S3) and the culturing in the second medium (S4).

When artificial skin is formed according to the aforementioned method, melanocytes are uniformly distributed on the epidermis basal layer, their distribution density is substantially consistent on a parallel surface to the surface of a dermis replacement, and thus an artificial skin model that is substantially similar to real human skin may be obtained.

In other words, an artificial skin model wherein keratinocytes and melanocytes are effectively present may be realized by inducing differentiation of melanocyte stem cells present among keratinocytes without separately culturing the melanocytes.

Another embodiment of the present invention provides artificial skin including a dermis simulating layer and an epidermis simulating layer on the dermis simulating layer, wherein the epidermis simulating layer includes an epidermis basal layer part being adjacent to the dermis simulating layer and a horny layer part exposed to the air, and keratinocytes present in the horny layer and melanocytes present in the epidermis basal layer originate from the same person.

The origination of the keratinocytes and the melanocytes of the artificial skin from the same person means that the melanocytes are not cultured separately from the keratinocytes but are differentiated from melanocyte stem cells present among keratinocytes during the production process of the artificial skin.

Yet another embodiment of the present invention provides a method of evaluating an effect of a pigmentation controlling agent including applying the pigmentation controlling agent to the artificial skin or artificial skin obtained according to the aforementioned production method, and then comparing a pigmentation degree of the artificial skin to which the pigmentation controlling agent is applied with a pigmentation degree of artificial skin to which the pigmentation controlling agent is not applied.

The term "pigmentation controlling" indicates changing, for example, directly or indirectly increasing or decreasing or suppressing a compositional or inductive pigmentation level of skin. The pigmentation controlling agent may be a pigmentation-increasing or decreasing agent.

The pigmentation controlling agent may be applied to the artificial skin through direct contact. The pigmentation controlling agent may be, for example, whitening cosmetics, but is not limited thereto.

Hereinafter, the present disclosure is illustrated in more detail with reference to examples. However, these examples are exemplary, and the present disclosure is not limited thereto.

### Production of Artificial Skin

### Example 1

### (1) Step 1: Production of Dermis Simulating Layer

Fibroblasts were mixed with collagen to form a dermis simulating layer, and then the dermis simulating layer was cultured for one week.

### (2) Step 2: Keratinocyte Application and Culture

After the 1^{st} step, keratinocytes (HEKn, Thermo Fisher Scientific Company) were applied on the dermis simulating layer and then cultured for 2 days.

### (3) Step 3: Culture in First Medium

After the 2^{nd} step, air culturing proceeded for 8 days by disposing a first medium (CnT-3D-PR, CellnTEC) to contact a bottom part of the reconstructed dermis, while an epidermis layer was exposed to the air.

### (4) Step 4: Culture in Second Medium

After the 3^{rd} step, the air culturing additionally proceeded for 6 days in a second medium to obtain an artificial skin model. The second medium was prepared by mixing a DMED medium (LM001-05, Welgene Inc.) and an F12 medium (LM010-01, Welgene Inc.) in a ratio of 2.5:1 and adding hydrocortisone, triiodothyronine, cholera toxin, ascorbic acid, and calcium chloride (CaCl₂) thereto.

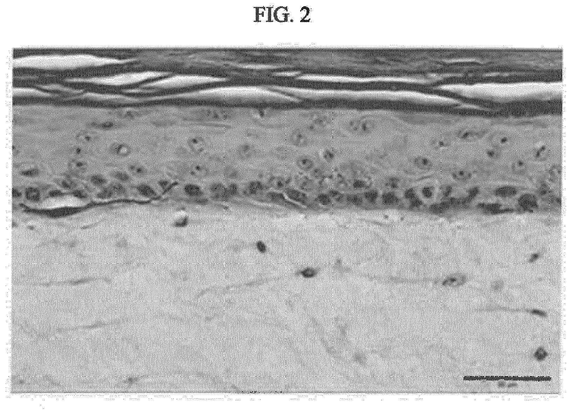

FIG. 2 is a microscope photograph showing a cross-sectional view of the artificial skin model.

### Comparative Example 1

Artificial skin was prepared according to the same method as Example 1, except for not performing the culturing in the second medium (the 4^{th} step).

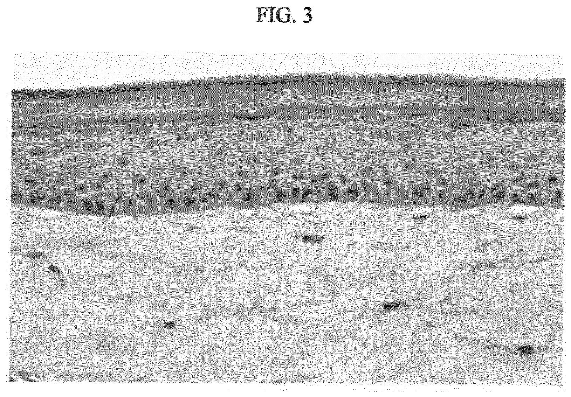

FIG. 3 is a microscope photograph showing a cross-sectional view of the artificial skin model.

### Evaluation: Cross Section Examination of Artificial Skin Model

The cross-sections of the artificial skin models according to Example 1 and Comparative Example 1 were examined with a microscope.

FIGS. 2 and 3 are microscope photographs showing the cross-sections of the artificial skins according to Example 1 and Comparative Example 1 after dying an H&E tissue.

Referring to FIGS. 2 and 3, a pigmentated artificial skin was obtained by using a keratinocyte alone and adjusting a medium for culturing artificial skin. In detail, the same keratinocytes were used, but melanocytes differentiated from melanocyte stem cells in the base of the epidermis layer were found in Example 1 of the present invention (FIG. 2), while melanocytes were not found in the base of the epidermis layer in Comparative Example 1 (FIG. 3).

## Claims

1. A method of producing artificial skin, comprising:
mixing fibroblasts and collagen to form a dermis simulating layer;
applying keratinocytes including melanocyte stem cells on the dermis simulating layer and culturing the same;
culturing the dermis simulating layer in a first medium that is a keratinocyte medium; and culturing the dermis simulating layer that is cultured in the first medium in a second medium including a DMEM medium and an F12 medium,
wherein the keratinocyte medium includes a bovine pituitary extract (BPE), a human epidermal growth factor (hEGF), bovine insulin, hydrocortisone, gentamicin, and amphotericin-B (GA-1000).

2. The method of claim 1, wherein the second medium includes a component inducing differentiation of the melanocyte stem cells.

3. The method of claim 1, wherein the first medium is disposed to contact a bottom surface of the dermis simulating layer, while the opposite surface thereof where the first medium is disposed is exposed to the air.

4. The method of claim 1, wherein the culturing in the second medium proceeds directly after the culturing in the first medium.

5. The method of claim 1, wherein the culturing in the first medium and the culturing in the second medium proceed within about 20 days in total.

6. The method of claim 5, wherein the culturing period in the first medium is equal to or longer than the culturing period in the second medium.

7. The method of claim 1, wherein the second medium is prepared by mixing the DMEM medium and the F12 medium in a ratio ranging from about 1:1 to about 4:1.

8. The method of claim 1, wherein the second medium further comprises at least one selected from hydrocortisone, triiodothyronine, cholera toxin, ascorbic acid, and calcium chloride (CaCl₂).

9. The method of claim 1, wherein the culturing in the second medium proceeds in an exposed state to the air.

10. Artificial skin comprising a dermis simulating layer and an epidermis simulating layer on the dermis simulating layer,
wherein the epidermis simulating layer comprises an epidermis basal layer part being adjacent to the dermis simulating layer and a horny layer part exposed to the air, and
keratinocytes present in the horny layer and melanocytes present in the epidermis basal layer originate from the same person.

11. An effect evaluation method of a pigmentation controlling agent, comprising:
applying the pigmentation controlling agent to artificial skin produced according to any one of claims 1 to 9 or artificial skin according to claim 10; and
comparing a pigmentation degree of the artificial skin to which the pigmentation controlling agent is applied with a pigmentation degree of artificial skin to which the pigmentation controlling agent is not applied.

12. The effect evaluation method of claim 11, wherein the pigmentation controlling agent is applied through direct contact with the artificial skin.

## Patentansprüche

1. Verfahren zum Herstellen künstlicher Haut, umfassend:
Mischen von Fibroblasten und Kollagen zur Bildung einer die Dermis simulierenden Schicht,
Aufbringen von Keratinozyten, einschließlich Melanozyten-Stammzellen, auf die die Dermis simulierende Schicht und Kultivieren derselben,
Kultivieren der die Dermis simulierenden Schicht in einem ersten Medium, das ein Keratinozytenmedium ist,
und Kultivieren der die Dermis simulierenden Schicht, die in dem ersten Medium kultiviert wird, in einem zweiten Medium, das ein DMEM-Medium und ein F12-Medium enthält,
wobei das Keratinozytenmedium einen Rinderhypophysenextrakt (BPE), einen humanen epidermalen Wachstumsfaktor (hEGF), Rinderinsulin, Hydrocortison, Gentamicin und Amphotericin-B (GA-1000) enthält.

2. Verfahren nach Anspruch 1, wobei das zweite Medium eine Komponente enthält, welche die Differenzierung der Melanozyten-Stammzellen induziert.

3. Verfahren nach Anspruch 1, wobei das erste Medium so angeordnet wird, dass es mit einer unteren Oberfläche der die Dermis simulierenden Schicht in Kontakt kommt, während die gegenüberliegende Oberfläche davon, wo das erste Medium angeordnet ist, der Luft ausgesetzt ist.

4. Verfahren nach Anspruch 1, wobei das Kultivieren in dem zweiten Medium direkt nach dem Kultivieren in dem ersten Medium abläuft.

5. Verfahren nach Anspruch 1, wobei das Kultivieren in dem ersten Medium und das Kultivieren in dem zweiten Medium innerhalb von insgesamt etwa 20 Tagen erfolgen.

6. Verfahren nach Anspruch 5, wobei die Kultivierungsdauer im ersten Medium gleich oder länger als die Kultivierungsdauer im zweiten Medium ist.

7. Verfahren nach Anspruch 1, wobei das zweite Medium durch Mischen des DMEM-Mediums und des F12-Mediums in einem Verhältnis im Bereich von etwa 1:1 bis etwa 4:1 hergestellt wird.

8. Verfahren nach Anspruch 1, wobei das zweite Medium ferner mindestens eines, das aus Hydrocortison, Triiodthyronin, Choleratoxin, Ascorbinsäure und Calciumchlorid (CaCl₂) ausgewählt ist, umfasst.

9. Verfahren nach Anspruch 1, wobei das Kultivieren im zweiten Medium in einem der Luft ausgesetzten Zustand erfolgt.

10. Künstliche Haut, die eine die Dermis simulierende Schicht und eine die Epidermis simulierende Schicht auf der die Dermis simulierenden Schicht umfasst,
wobei die die Epidermis simulierende Schicht einen Epidermis-Basallagenteil, der an die die Dermis simulierende Schicht angrenzt, und einen der Luft ausgesetzten Hornschichtteil umfasst, und
Keratinozyten, die in der Hornschicht vorliegen und Melanozyten, die in der Epidermis-Basallage vorliegen, von derselben Person stammen.

11. Verfahren zur Bewertung der Wirkung eines die Pigmentierung kontrollierenden Mittels, umfassend:
Auftragen des die Pigmentierung kontrollierenden Mittels auf künstliche Haut, die gemäß einem der Ansprüche 1 bis 9 hergestellt wurde, oder auf künstliche Haut gemäß Anspruch 10 und
Vergleichen eines Pigmentierungsgrades der künstlichen Haut, auf die das die Pigmentierung kontrollierende Mittel aufgetragen wird, mit einem Pigmentierungsgrad von künstlicher Haut, auf die das die Pigmentierung kontrollierende Mittel nicht aufgetragen wird.

12. Verfahren zur Bewertung der Wirkung nach Anspruch 11, wobei das die Pigmentierung kontrollierende Mittel durch direkten Kontakt mit der künstlichen Haut aufgetragen wird.

## Revendications

1. Procédé de production de peau artificielle, comprenant :
le mélange de fibroblastes et de collagène pour former une couche de simulation de derme ;
l'application de kératinocytes comprenant des cellules souches de mélanocytes sur la couche de simulation de derme et leur mise en culture ;
la mise en culture de la couche de simulation de derme dans un premier milieu qui est un milieu pour kératinocytes ; et la mise en culture de la couche de simulation de derme qui est mise en culture dans le premier milieu dans un deuxième milieu comprenant un milieu DMEM et un milieu F12,
dans lequel le milieu pour kératinocytes comprend un extrait pituitaire bovin (BPE), un facteur de croissance épidermique humain (hEGF), de l'insuline bovine, de l'hydrocortisone, de la gentamicine, et de l'amphotéricine-B (GA-1000).

2. Procédé selon la revendication 1, dans lequel le deuxième milieu comprend un composant induisant la différenciation des cellules souches de mélanocytes.

3. Procédé selon la revendication 1, dans lequel le premier milieu est disposé pour être en contact avec une surface de dessous de la couche de simulation de derme, tandis que la surface opposée à celle où est disposé le premier milieu est exposée à l'air.

4. Procédé selon la revendication 1, dans lequel la mise en culture dans le deuxième milieu s'effectue directement après la mise en culture dans le premier milieu.

5. Procédé selon la revendication 1, dans lequel la mise en culture dans le premier milieu et la mise en culture dans le deuxième milieu s'effectuent dans un délai d'environ 20 jours au total.

6. Procédé selon la revendication 5, dans lequel la période de mise en culture dans le premier milieu est supérieure ou égale à la période de mise en culture dans le deuxième milieu.

7. Procédé selon la revendication 1, dans lequel le deuxième milieu est préparé par mélange du milieu DMEM et du milieu F12 dans un rapport allant d'environ 1:1 à environ 4:1.

8. Procédé selon la revendication 1, dans lequel le deuxième milieu comprend en outre au moins l'un choisi parmi l'hydrocortisone, la triiodothyronine, la toxine cholérique, l'acide ascorbique, et le chlorure de calcium (CaCl₂).

9. Procédé selon la revendication 1, dans lequel la mise en culture dans le deuxième milieu s'effectue dans un état exposé à l'air.

10. Peau artificielle comprenant une couche de simulation de derme et une couche de simulation d'épiderme sur la couche de simulation de derme,
dans laquelle la couche de simulation d'épiderme comprend une couche basale d'épiderme qui est en partie adjacente à la couche de simulation de derme et une partie de couche cornée exposée à l'air, et
les kératinocytes présents dans la couche cornée et les mélanocytes présents dans la couche basale d'épiderme proviennent de la même personne.

11. Procédé d'évaluation d'effet d'un agent de régulation de pigmentation, comprenant :
l'application de l'agent de régulation de pigmentation sur une peau artificielle produite selon l'une quelconque des revendications 1 à 9 ou une peau artificielle selon la revendication 10 ; et
la comparaison d'un degré de pigmentation de la peau artificielle sur laquelle l'agent de régulation de pigmentation est appliqué à un degré de pigmentation de peau artificielle sur laquelle l'agent de régulation de pigmentation n'est pas appliqué.

12. Procédé d'évaluation d'effet selon la revendication 11, dans lequel l'agent de régulation de pigmentation est appliqué par contact direct avec la peau artificielle.
